# EUROPÄISCHE PATENTSCHRIFT

(11) **EP 1 233 712 B1**
(45) Veröffentlichungstag und Bekanntmachung des Hinweises auf die Patenterteilung: **25.02.2004**
(21) Anmeldenummer: 99974233.1
(22) Anmeldetag: 03.12.1999
(51) Int. Cl.: A61B 17/74

(54) **INTRAMEDULLÄRER MARKNAGEL**
INTRAMEDULLARY NAIL
CLOU INTRAMEDULLAIRE

(43) Veröffentlichungstag der Anmeldung: 28.08.2002
(73) Patentinhaber: SYNTHES AG Chur, 7002 Chur (CH)
(72) Erfinder: FRIGG, Robert, CH-2544 Bettlach (CH); BRUNNER, Peter, CH-3008 Bern (CH)
(74) Vertreter: Lusuardi, Werther Giovanni, Dr.
(86) Internationale Anmeldenummer: PCT/CH1999/000581
(87) Internationale Veröffentlichungsnummer: WO 2001/039679

(56) Entgegenhaltungen:
- EP-A- 0 551 588
- US-A- 3 433 220

## Beschreibung

Die Erfindung betrifft einen intramedullären Marknagel gemäss dem Oberbegriff des Patentanspruchs 1.

Aus der EP-A 0 551 588 ist ein derartiger Marknagel bekannt. Die Nachteile dieser Anordnung bestehen darin, dass die beiden Hüftschrauben, welche in den Femurkopf eingesetzt werden (Hüftkopfschraube und Hüftpin), winkelstabil in den Bohrungen des Marknagels geführt sind. Durch ihre, dimensionsbedingte, unterschiedliche Biegesteifigkeit kann es in der Klinik vorkommen, dass sich die beiden Schrauben relativ zueinander verschieben. Durch die schlechte oder gar blockierte axiale Gleiteigenschaft des kleineren Hüftpins, kann dieser das Hüftgelenk penetrieren.

Hier will die Erfindung Abhilfe schaffen. Der Erfindung liegt das Problem zugrunde, einen intramedullären Marknagel zu schaffen, der den oben beschriebenen Nachteil nicht aufweist.

Die Erfindung löst die gestellte Aufgabe mit einem intramedullären Marknagel, welcher die Merkmale des Anspruchs 1 aufweist.

Der durch das Langloch bewirkte Verzicht auf die Winkelstabilität in Richtung der Nagellängsachse des Hüftpins bedeutet keinen Nachteil gegenüber dem Stand der Technik, da die Rotation des Hüftkopfes rund um die Hüftkopfschraube mittels des Hüftpins noch immer verhindert wird, was ja die eigentliche Aufgabe des Hüftpins ist und bleibt.
Durch Verwendung eines Langlochs, zur Aufnahme des Hüftpins, ist der Vorteil erzielbar, dass die mechanischen Beanspruchungen der beiden Hüftschrauben unterteilt werden kann. Die grössere Hüftkopfschraube übernimmt dabei die Funktion der Winkelstabilität der Schraubenverankerung im Hüftkopf, relativ zum Marknagel und erlaubt ein "Sintern" des Femurkopffragmentes durch axiales Gleiten in der entsprechenden Marknagelbohrung. Der kleinere Hüftpin übernimmt dabei nur noch die Rotationssicherung des Femurkopfes, relativ zur grösseren Hüftkopfschraube.

Bei einer bevorzugten Ausführungsform der Erfindung ist die erste Bohrung kreiszylindrisch ausgebildet. Die laterale Eingangsöffnung der zweiten Bohrung ist vorzugsweise kreiszylindrisch und die mediale Austrittsöffnung der zweiten Bohrung vorzugsweise als Langloch ausgebildet.
Bei einer anderen Ausführungsform kann sowohl die laterale Eingangsöffnung als auch die mediale Austrittsöffnung der zweiten Bohrung als Langloch ausgebildet sein.
Die Breite B der zweiten Bohrung ist vorzugsweise kleiner als der Durchmesser D der ersten Bohrung.

Die Mittelachse der ersten Bohrung schliesst vorzugsweise einen Winkel α von 40° - 55° zur Längsachse ein.
Die Mittelachse der zweiten Bohrung schliesst vorzugsweise einen Winkel β von 30° bis 70° zur Längsachse ein, bzw. einen Winkel von 180°- β von 110° bis 150°. Diese Stellung ist vorzugsweise für die Verwendung einer nicht-gleitenden Verankerungsschraube vorgesehen.
Der Winkel β kann aber auch einen Wert von 70° bis 90° aufweisen, sofern der Winkel α im gleichen Winkelbereich liegt. Diese Stellung ist vorzugsweise für die Verwendung einer Gleithüftschraube vorgesehen.
Die Länge L des Längsloches beträgt vorzugsweise 8 bis 12 mm, typischerweise 9 bis 11 mm. Die Breite B des Längsloches beträgt vorzugsweise 5 bis 10 mm, typischerweise 6 bis 7 mm.
Das Verhältnis L:B liegt vorzugsweise im Bereich von 1,05 bis 2,00, typischerweise im Bereich von 1,10 bis 1,60.

Bei einer bevorzugten Ausführungsform ist zusätzlich mindestens eine, näher beim distalen Ende gelegene, die Längsachse quer schneidende, dritte Bohrung, zur Aufnahme einer distalen Verriegelungsschraube, vorgesehen.

Bei einer weiteren Ausführungsform kann die laterale Eingangsöffnung der zweiten Bohrung als Langloch ausgebildet sein und die mediale Austrittsöffnung der zweiten Bohrung kreiszylindrisch ausgebildet sein.

Der innerhalb einer Fixationsvorrichtung zusammen mit dem Marknagel zu verwendende Hüftpin sollte vorzugsweise 5 bis 20 mm, typischerweise 10 bis 15 mm kürzer sein als die zu verwendende Hüftkopfschraube. Der Durchmesser des Hüftpins sollte dabei vorzugsweise der Breite B der als Langloch ausgebildeten zweiten Bohrung entsprechen.

Die Erfindung und Weiterbildungen der Erfindung werden im folgenden anhand der teilweise schematischen Darstellung eines Ausführungsbeispiels noch näher erläutert.

Es zeigen:
Fig. 1 einen Längsschnitt durch den erfindungsgemässen Marknagel mit dazu geeigneten Hüft- und Verriegelungsschrauben in Explosionsdarstellung;
Fig. 2 eine um 90° verdrehte Ansicht des proximalen Abschnitts des Marknagels gemäss Fig. 1;
Fig. 3 eine Modifikation des proximalen Abschnitts des Marknagels gemäss Fig. 1;
Fig. 4 eine perspektivischen Darstellung des in den Femur implantierten und verriegelten Marknagels gemäss Fig. 1;
Fig. 5 eine laterale Ansicht des proximalen Abschnitts des Marknagels mit einem stark gerundeten Langloch;
Fig. 6 eine laterale Ansicht des proximalen Abschnitts des Marknagels mit einem rechteckigen Langloch;
Fig. 7 eine laterale Ansicht des proximalen Abschnitts des Marknagels mit einem leicht gerundeten Langloch;
Fig. 8 einen Schnitt durch den proximalen Abschnitts nach Fig. 3 mit einem sich latero-medial erweiternden, nach proximal geneigten Langloch;
Fig. 9 einen Schnitt durch einen proximalen Abschnitt eines Marknagels mit einem sich latero-medial erweiternden Langloch; und
Fig. 10 einen Schnitt durch einen proximalen Abschnitt eines Marknagels mit einem sich latero-medial verengenden Langloch.

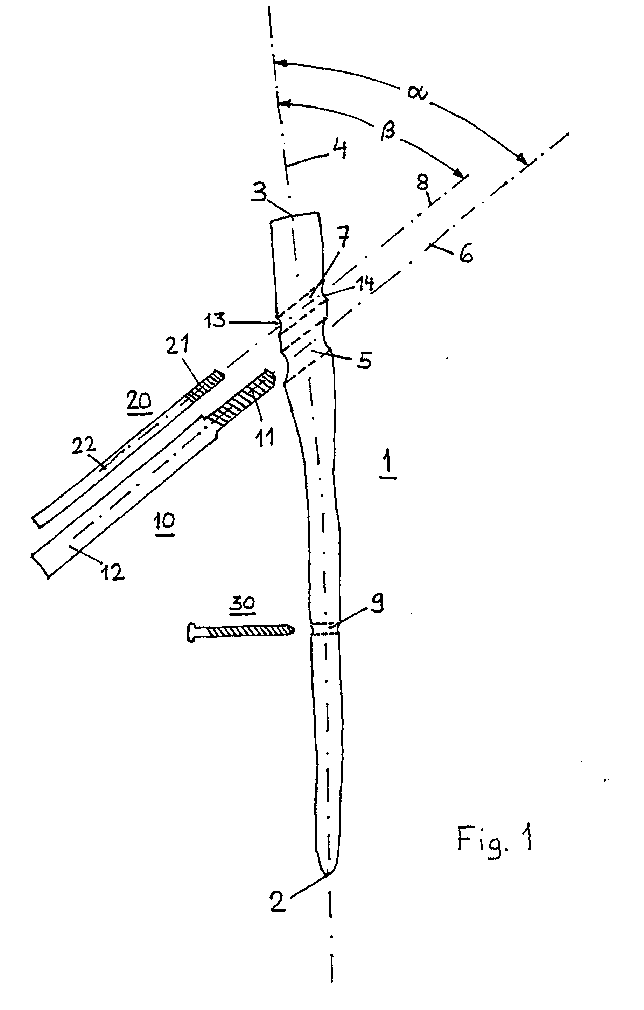

Der in Fig. 1 und 4 dargestellte intramedullärer Marknagel 1 zur Versorgung von Femurfrakturen weist ein zur Einführung in den Markraumkanal bestimmtes, distales Ende 2, ein proximales Ende 3 und eine Längsachse 4 auf.
Der Marknagel 1 weist ferner eine näher beim proximalen Ende 3 gelegene, die Längsachse 4 quer schneidende erste Bohrung 5 mit der Längsachse 6 auf, die zur Aufnahme einer hüftkopfschraube 10 bestimmt ist. Die Mittelachse 6 der ersten kreiszylindrischen Bohrung 5 weist einen Winkel α von 30° bis 70° zur Längsachse 4 auf, bzw. einen Winkel 180°- α von 110° bis 150°.
Im weiteren ist der Marknagel 1 mit einer zwischen der ersten Bohrung 5 und dem proximalen Ende 3 gelegenen, die Längsachse 4 quer schneidenden zweiten Bohrung 7 mit der Längsachse 8 zur Aufnahme eines Hüftpins 20 versehen.
Die Mittelachse der zweiten Bohrung schliesst einen Winkel ß von 45° zur Längsachse ein.

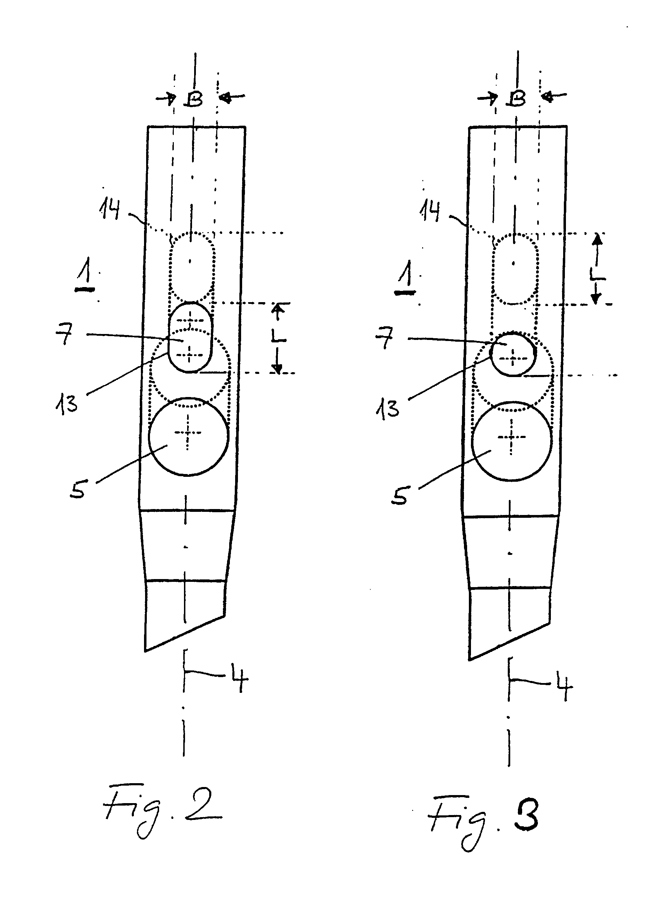

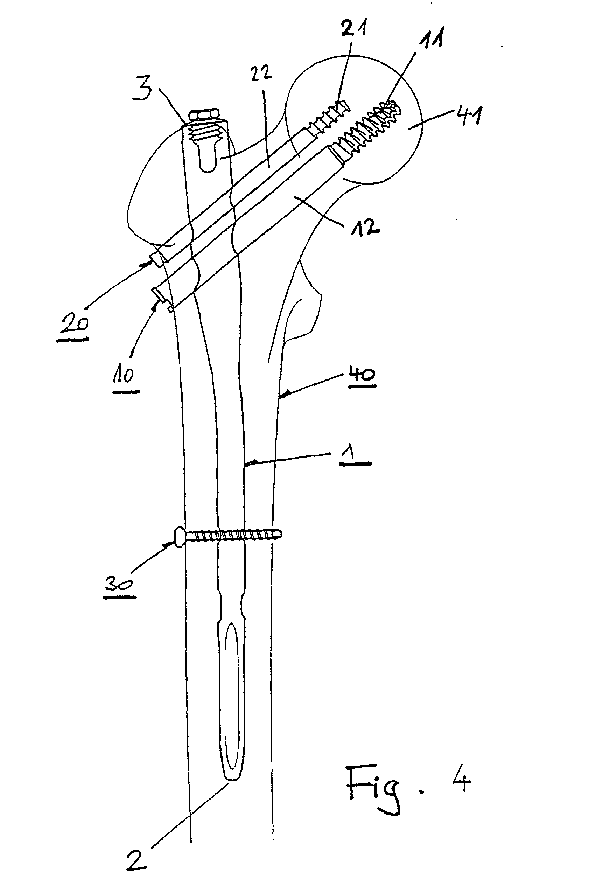

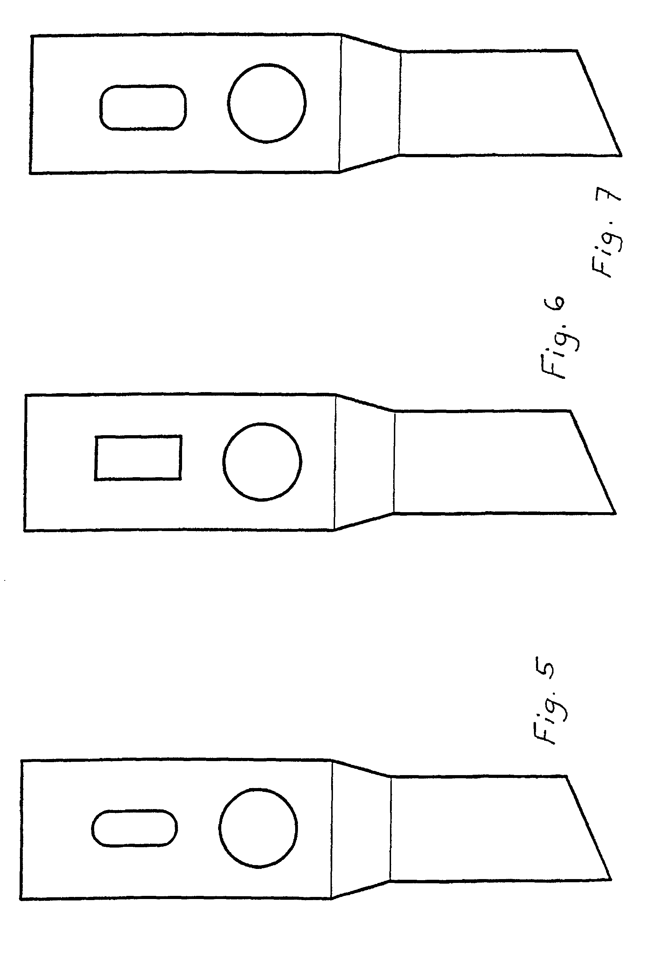

Wie in Fig. 2 dargestellt ist sowohl die laterale Eingangsöffnung 13 als auch die mediale Austrittsöffnung 14 der zweiten Bohrung 7 als Langloch ausgebildet. Bei dieser Ausführungsform kann der in die Bohrung 7 einzuführende Hüftpin 20 in der latero-medialen Ebene (diese entspricht der Zeichenebene in Fig. 1) gegenüber der Achse 8 in beiden Richtungen, d.h. nach oben und unten, etwas abgewinkelt werden.

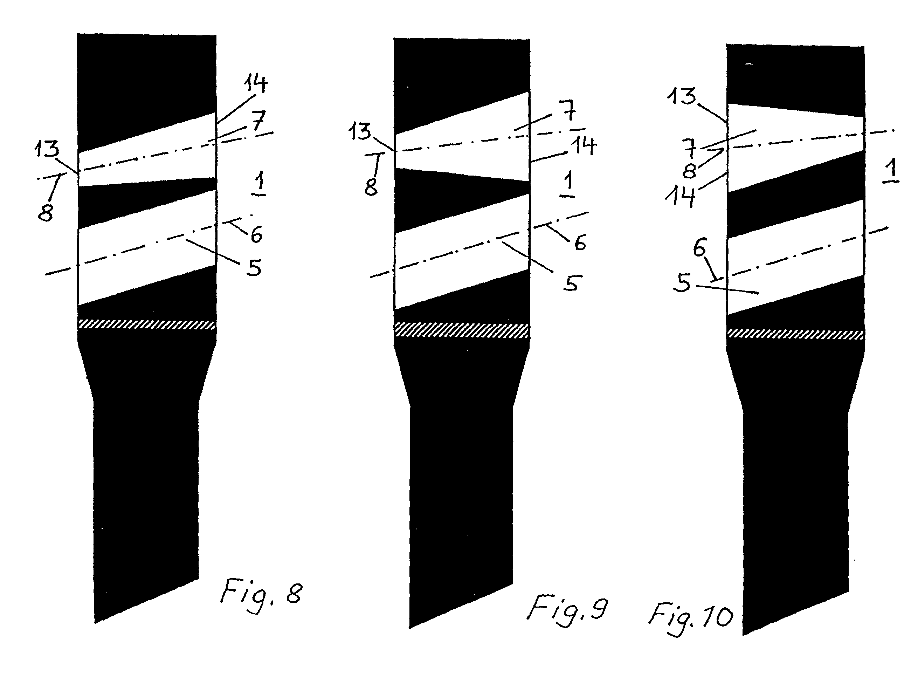

Wie in Fig. 3 (sowie den Fig. 8 und 9) dargestellt, kann die zweite Bohrung 7 aber auch so gestaltet sein, dass ihre laterale Eingangsöffnung 13 kreiszylindrisch ist und die mediale Austrittsöffnung 14 als Langloch ausgebildet ist. Bei dieser Ausführungsform wird der in die Bohrung 7 einzuführende Hüftpin 20 an der lateralen Eingangsöffnung 13 vollständig umschlossen, so dass dieser nur noch eine beschränkte Bewegung in der latero-medialen Ebene (diese entspricht der Zeichenebene in Fig. 1) gegenüber der Achse 8 ausführen kann.

Das Langloch kann wie in den Figuren 2, 3, 5 und 7 dargestellt abgerundet sein (diese Form ergibt sich bei der Herstellung der Bohrung mittels eines kreiszylindrischen Bohrers oder Fräsers der quer zur Bohrerachse bewegt wird) oder aber auch - wie in Fig. 6 dargestellt - eckig in Form eines Rechtecks ausgebildet sein. Unabhängig von der mehr rechteckigen oder abgerundeten Ausführung des Langloches besitzt dieses eine Breite B und eine Länge L > B wobei die Länge L des Langlochs in Richtung der Längsachse 4 verläuft. Vorzugsweise ist die Breite B der zweiten Bohrung 7 kleiner als der Durchmesser D der ersten Bohrung 5. Die Länge L des Längsloches beträgt 10 mm, seine Breite 6,5 mm. Das Verhältnis zwischen L:B liegt somit bei 1,538.

Wie in den Fig. 1 und 4 dargestellt weist der Marknagel 1 eine zusätzliche, näher beim distalen Ende 2 gelegene, die Längsachse 4 quer schneidende, dritte Bohrung 9 auf, zur Aufnahme einer distalen Verriegelungsschraube 30.

Wie in Fig. 4 dargestellt, ist es vorteilhaft, wenn der Hüftpin 20, der aus dem Schaft 22 und dem gewindeten Vorderteil 21 besteht, 5 bis 20 mm, vorzugsweise 10 bis 15 mm kürzer als die Hüftkopfschraube 10 ist, welche aus dem Schaft 12 und dem gewindeten Vorderteil 11 besteht.

Im folgenden wird kurz die Operationstechnik des erfindungsgemässen Marknagels beschrieben:
a) Vorbereitung des Markraumkanals des Femur;
b) Einschlagen des Marknagels in den Markraumkanal;
c) Einführung von Führungsdrähten in die erste proximale Bohrung 6 und in die zweite proximale Bohrung 7;
d) Bohrung des Loches für den Hüftpin 20 im Knochen;
e) Einführung des Hüftpins 20 durch die zweite proximale Bohrung 7 in des vorbereitete Loch im Knochen; nach erfolgter Einführung des Hüftpins 20 ist der Hüftkopf gegen Rotation bei der nachfolgenden Einführung der Hüftkopfschraube 10 gesichert;
f) Bohrung des Loches für die Hüftkopfschraube 10 im Knochen;
g) Einführung der Hüftkopfschraube 10 durch die erste proximale Bohrung 5 in das vorbereitete Loch im Knochen und Entfernung des Führungsdrahtes;
h) Bohrung des Loches für die distale Verriegelung des Marknagels; Einführung einer Verriegelungsschraube 30 in die distale gelegene, dritte Bohrung 9.

## Patentansprüche

1. Fixationsvorrichtung zur Versorgung von Femurfrakturen mit einem intramedullären Marknagel (1), einer Hüftkopfschraube (10) und einem Hüftpin (20), wobei
A) der intramedulläre Marknagel (1) ein zur Einführung in den Markraumkanal bestimmtes, distales Ende (2), ein proximales Ende (3), eine Längsachse (4) sowie eine näher beim proximalen Ende (3) gelegene, die Längsachse (4) quer schneidende erste Bohrung (5) zur Aufnahme der Hüftkopfschraube (10) aufweist, wobei die Mittelachse (6) der ersten Bohrung (5) einen Winkel α von 30° bis 70° zur Längsachse (4) einschliesst,
B) der intramedulläre Marknagel (1) eine zwischen der ersten Bohrung (5) und dem proximalen Ende (3) gelegene, die Längsachse (4) quer schneidende zweite Bohrung (7) zur Aufnahme des Hüftpins (20) aufweist, wobei die zweite Bohrung (7) mindestens teilweise als Langloch ausgebildet ist mit einer Breite B und einer Länge L > B, **dadurch gekennzeichnet, dass** die Länge L des Langlochs in Richtung der Längsachse (4) verläuft.

2. Fixationsvorrichtung nach Anspruch 1, **dadurch gekennzeichnet, dass** die erste Bohrung (5) kreiszylindrisch ist.

3. Fixationsvorrichtung nach Anspruch 1 oder 2, **dadurch gekennzeichnet, dass** die laterale Eingangsöffnung (13) der zweiten Bohrung (7) kreiszylindrisch und die mediale Austrittsöffnung (14) der zweiten Bohrung (7) als Langloch ausgebildet ist.

4. Fixationsvorrichtung nach Anspruch 1 oder 2, **dadurch gekennzeichnet, dass** sowohl die laterale Eingangsöffnung (13) als auch die mediale Austrittsöffnung (14) der zweiten Bohrung (7) als Langloch ausgebildet ist.

5. Fixationsvorrichtung nach einem der Ansprüche 1 bis 3, **dadurch gekennzeichnet, dass** die Breite B der zweiten Bohrung (7) kleiner ist als der Durchmesser D der ersten Bohrung (5).

6. Fixationsvorrichtung nach einem der Ansprüche 1 bis 5, **dadurch gekennzeichnet, dass** die Mittelachse (8) der zweiten Bohrung (7) einen Winkel β von 30° bis 70° zur Längsachse (4) einschliesst.

7. Fixationsvorrichtung nach einem der Ansprüche 1 bis 5, **dadurch gekennzeichnet, dass** die Mittelachse (8) der zweiten Bohrung (7) einen Winkel β von 70° bis 90° zur Längsachse (4) einschliesst.

8. Fixationsvorrichtung nach einem der Ansprüche 1 bis 7, **dadurch gekennzeichnet, dass** die Länge L des Längsloches 8 bis 12 mm, vorzugsweise 9 bis 11 mm beträgt.

9. Fixationsvorrichtung nach einem der Ansprüche 1 bis 7, **dadurch gekennzeichnet, dass** die Breite B des Längsloches 5 bis 10 mm, vorzugsweise 6 bis 7 mm beträgt.

10. Fixationsvorrichtung nach einem der Ansprüche 1 bis 9, **dadurch gekennzeichnet, dass** zusätzlich mindestens eine, näher beim distalen Ende (2) gelegene, die Längsachse (4) quer schneidende, dritte Bohrung (9), zur Aufnahme einer distalen Verriegelungsschraube (30), vorgesehen ist.

11. Fixationsvorrichtung nach einem der Ansprüche 1 bis 10, **dadurch gekennzeichnet, dass** die Mittelachse (6) der ersten Bohrung (5) einen Winkel α von 40° - 55° zur Längsachse (4) einschliesst.

12. Fixationsvorrichtung nach einem der Ansprüche 1 bis 11, **dadurch gekennzeichnet, dass** das Verhältnis L:B im Bereich von 1,05 bis 2,00, vorzugsweise von 1,10 bis 1,60 liegt.

13. Fixationsvorrichtung nach einem der Ansprüche 1 bis 12, **dadurch gekennzeichnet, dass** die laterale Eingangsöffnung (13) der zweiten Bohrung (7) Langloch ausgebildet ist und die mediale Austrittsöffnung (14) der zweiten Bohrung (7) kreiszylindrisch ausgebildet ist.

14. Fixationsvorrichtung nach einem der Ansprüche 1 bis 13, **dadurch gekennzeichnet, dass** der Hüftpin (20) 5 bis 20 mm, vorzugsweise 10 bis 15 mm kürzer als die Hüftkopfschraube (10) ist.

15. Fixationsvorrichtung nach Anspruch 14, **dadurch gekennzeichnet, dass** der Durchmesser des Hüftpins (20) der Breite B der als Langloch ausgebildeten zweiten Bohrung (7) entspricht.

## Claims

1. A fixation device for fixating femoral fractures including an intramedullary nail (1), a femur head screw (10) and a femur head pin (20), whereby
A) the intramedullary nail (1) comprises a distal end (2) adapted to be introduced into the medullary canal, a proximal end (3), a longitudinal axis (4), as well as a first bore (5) for receiving a femur head screw (10) situated closer to the proximal end (3) and obliquely intersecting said longitudinal axis (4), the central axis (6) of said first bore (5) forming an angle α of between 30 and 70 degrees relative to said longitudinal axis (4),
B) the intramedullary nail (1) comprises a bore (7) for receiving the femur head pin (20) situated between the first bore (5) and the proximal end (3) and obliquely intersecting said longitudinal axis (4), whereby the second bore (7) is at least partially shaped in the form of an elongate hole with a width B and a length L > B,
charecterised in that
the length L of the elongate hole is extending in the direction of said longitudinal axis (4).

2. A fixation device as claimed in claim 1, **characterised in that** the first bore (5) is circularly cylindrical.

3. A fixation device as claimed in claim 1 or 2, **characterised in that** the lateral entrance opening (13) of the second bore (7) is circularly cylindrical and that the medial exit opening (14) of the second bore (7) is shaped in the form of an elongate hole.

4. A fixation device as claimed in claim 1 or 2, **characterised in that** both the lateral entrance opening (13) and the medial exit opening (14) of the second bore (7) are shaped in the form of an elongate hole.

5. A fixation device as claimed in any of the claims 1 to 3, **characterised in that** the width B of the second bore (7) is smaller than the diameter D of the first bore (5).

6. A fixation device as claimed in any of the claims 1 to 5, **characterised in that** the central axis (8) of the second bore (7) forms an angle β of between 30 and 70 degrees relative to the longitudinal axis (4).

7. A fixation device as claimed in any of the claims 1 to 5, **characterised in that** the central axis (8) of the second bore (7) forms an angle β of between 70 and 90 degrees relative to the longitudinal axis (4).

8. A fixation device as claimed in any of the claims 1 to 7, **characterised in that** the length L of the elongate hole is between 8 and 12 mm, preferably between 9 and 11 mm.

9. A fixation device as claimed in any of the claims 1 to 7, **characterised in that** the width B of the elongate hole is between 5 and 10 mm, preferably between 6 and 7 mm.

10. A fixation device as claimed in any of the claims 1 to 9, **characterised in that**, in addition, situated closer to the distal end (2), at least one third bore (9) is provided for receiving a distal locking screw (30).

11. A fixation device as claimed in any of the claims 1 to 10, **characterised in that** the central axis (6) of the first bore (5) forms an angle α of between 40 and 55 degrees relative to the longitudinal axis (4).

12. A fixation device as claimed in any of the claims 1 to 11, **characterised in that** the ratio L:B is within a range between 1.05 and 2.00, preferably between 1.10 and 1.60.

13. A fixation device as claimed in any of the claims 1 to 12, **characterised in that** the lateral entrance opening (13) of the second bore (7) is shaped in the form of an elongate hole and that the medial exit opening (14) of the second bore (7) is circularly cylindrical.

14. A fixation device as claimed in any of the claims 1 to 13, **characterised in that** the femur head pin (20) is shorter by 5 mm to 20 mm, preferably by 10 mm to 15 mm, than the femur head screw (10).

15. A fixation device as claimed in claim 14, **characterised in that** the diameter of the femur head pin (20) corresponds to the width B of the second bore (7) shaped in the form of an elongate hole.

## Revendications

1. Dispositif de fixation pour le traitement de fractures du fémur, avec un clou intramédullaire (1), une vis (10) de tête iliaque et un goujon iliaque (20), dans lequel
A) le clou intramédullaire (1) présente une extrémité distale (2) destinée être insérée dans le canal médullaire, une extrémité proximale (3), un axe longitudinal (4) ainsi qu'un premier alésage (5) de réception de la vis (10) de tête iliaque qui est situé à proximité de l'extrémité proximale (3) et qui coupe transversalement l'axe longitudinal (4), l'axe central (6) du premier alésage (5) formant avec l'axe longitudinal (4) un angle α compris entre 30° et 70°,
B) le clou intramédullaire (1) présente un deuxième alésage (7) de réception du goujon iliaque (20), qui coupe transversalement l'axe longitudinal (4) et qui est situé entre le premier alésage (5) et l'extrémité proximale (3), le deuxième alésage (7) étant au moins en partie configuré comme trou oblong d'une largeur B et d'une longueur L > B,
**caractérisé en ce que**
la longueur L du trou oblong s'étend dans la direction de l'axe longitudinal (4).

2. Dispositif de fixation selon la revendication 1, **caractérisé en ce que** le premier alésage (5) est cylindrique circulaire.

3. Dispositif de fixation selon les revendications 1 ou 2, **caractérisé en ce que** l'ouverture latérale d'entrée (13) du deuxième alésage (7) est cylindrique circulaire, l'ouverture médiale de sortie (14) du deuxième alésage (7) étant configurée comme trou oblong.

4. Dispositif de fixation selon les revendications 1 ou 2, **caractérisé en ce que** tant l'ouverture latérale d'entrée (13) que l'ouverture médiale de sortie (14) du deuxième alésage (7) sont configurées comme trous oblongs.

5. Dispositif de fixation selon l'une des revendications 1 à 3, **caractérisé en ce que** la largeur B du deuxième alésage (7) est inférieure au diamètre D du premier alésage (5).

6. Dispositif de fixation selon l'une des revendications 1 à 5, **caractérisé en ce que** l'axe central (8) du deuxième alésage (7) forme avec l'axe longitudinal (4) un angle β compris entre 30° et 70°.

7. Dispositif de fixation selon l'une des revendications 1 à 5, **caractérisé en ce que** l'axe central (8) du deuxième alésage (7) forme avec l'axe longitudinal (4) un angle β compris entre 70° et 90°.

8. Dispositif de fixation selon l'une des revendications 1 à 7, **caractérisé en ce que** la longueur L du trou oblong (8) peut atteindre 12 mm et est de préférence comprise entre 9 et 11 mm.

9. Dispositif de fixation selon l'une des revendications 1 à 7, **caractérisé en ce que** la largeur B du trou oblong (5) peut atteindre 10 mm et est de préférence comprise entre 6 et 7 mm.

10. Dispositif de fixation selon l'une des revendications 1 à 9, **caractérisé en ce que** de plus, au moins un troisième alésage (9) de réception d'une vis distale de verrouillage (30) est prévu à proximité de l'extrémité distale (2) et coupe transversalement l'axe longitudinal (4).

11. Dispositif de fixation selon l'une des revendications 1 à 10, **caractérisé en ce que** l'axe central (6) du premier alésage (5) forme avec l'axe longitudinal (4) un angle α compris entre 40° et 55°.

12. Dispositif de fixation selon l'une des revendications 1 à 11, **caractérisé en ce que** le rapport L:B est compris dans la plage de 1,05 à 2,00 et de préférence de 1,10 à 1,60.

13. Dispositif de fixation selon l'une des revendications 1 à 12, **caractérisé en ce que** l'ouverture latérale d'entrée (13) du deuxième alésage (7) est configurée comme trou oblong et **en ce que** l'ouverture médiale de sortie (14) du deuxième alésage (7) est cylindrique circulaire.

14. Dispositif de fixation selon l'une des revendications 1 à 13, **caractérisé en ce que** le goujon iliaque (20) est de 5 à 20 mm et de préférence de 10 à 15 mm plus court que la vis (10) de tête iliaque.

15. Dispositif de fixation selon la revendication 14, **caractérisé en ce que** le diamètre du goujon iliaque (20) correspond à la largeur B du deuxième alésage (7) configuré comme trou oblong.
